# EUROPEAN PATENT APPLICATION

(11) **EP 3 378 556 A1**
(43) Date of publication of application: **26.09.2018**
(21) Application number: 18161706.9
(22) Date of filing: 14.03.2018
(51) Int. Cl.: B01J 20/289, B01J 20/32, C07H 21/04

(54) **METHOD FOR THE PREPARATION OF A LOW UNSPECIFIC BINDING-SUPPORT FOR AFFINITY CHROMATOGRAPHY AND/OR ON-LINE SYNTHESIS OF OLIGONUCLEOTIDES**

(30) Priority: 20.03.2017 IT 201700030473
(71) Applicant: Platella, Chiara, 80132 Napoli (IT); Musumeci, Domenica, 80123 Napoli (IT); Amato, Jussara, 80131 Napoli (IT); Randazzo, Antonio, 67031 Castel di Sangro (AQ) (IT); Pagano, Bruno, 80131 Napoli (IT); Montesarchio, Daniela, 80128 Napoli (IT)
(72) Inventor: Platella, Chiara, 80132 Napoli (IT); Musumeci, Domenica, 80123 Napoli (IT); Amato, Jussara, 80131 Napoli (IT); Randazzo, Antonio, 67031 Castel di Sangro (AQ) (IT); Pagano, Bruno, 80131 Napoli (IT); Montesarchio, Daniela, 80128 Napoli (IT)
(74) Representative: Currado, Luisa

(57) **Abstract**

A method for preparing functionalized solid supports to be used in the on-line synthesis of support-bound, fully deprotected secondary structure-forming oligonucleotides and/or for low unspecific binding affinity chromatography is disclosed.

SEQUENCE LISTING
<110>  PLATELLA, Chiara
       MUSUMECI, Domenica
       AMATO, Jussara
       RANDAZZO, Antonio
       PAGANO, Bruno
       MONTESARCHIO, Daniela

<120>  Method for the preparation of a low unspecific binding-
support for affinity chromatography and/or on-line synthesis of
oligonucleotides
<130>  RBW14921-PCT
<150>  102017000030473
<151>  2017-03-20
<160>  2
<170>  PatentIn version 3.5
<210>  1
<211>  27
<212>  DNA
<213>  Synthetic
<400>  1
cgcgaattcg cgtttcgcga attcgcg                                          27
<210>  2
<211>  26
<212>  DNA
<213>  Synthetic
<400>  2
ttagggttag ggttagggtt agggtt                                           26

SEQUENCE LISTING

<110>  PLATELLA, Chiara
       MUSUMECI, Domenica
      AMATO, Jussara
      RANDAZZO, Antonio
      PAGANO, Bruno
      MONTESARCHIO, Daniela

<120>  Method for the preparation of a low unspecific binding-support
       for affinity chromatography and/or on-line synthesis of
       oligonucleotides

<130>  RBP14921-EP

<150>  102017000030473
<151>  2017-03-20

<160>  2

<170>  PatentIn version 3.5

<210>  1
<211>  27
<212>  DNA
<213>  Synthetic

<400>  1
cgcgaattcg cgtttcgcga attcgcg                                          27


<210>  2
<211>  26
<212>  DNA
<213>  Synthetic

<400>  2
ttagggttag ggttagggtt agggtt                                           26

## Description

### Background of the invention

The present invention refers to the chemical field since it relates to a method for preparing functionalized solid supports to be used for the on-line synthesis and deprotection of oligonucleotides anchored to a matrix of interest for affinity chromatography. More in particular, the present invention relates to a novel nucleoside-functionalized support which, due to its low-to-null unspecific binding, is suitable for extensive and quick affinity chromatography-based screenings of putative ligands of secondary structure-forming oligonucleotides.

### State of the art

The study of non-canonical DNA conformations, as G-quadruplex (G4) structures, is one of the hottest research fields in modern structural biology, particularly motivated by their crucial role in telomeres and gene promoters, with G4-forming sequences found in regulatory regions of humans, as well as in virus genomes as HIV, EBV and HSV-1. In addition to their increasing biological significance, the G4 structures are gaining interest in anticancer and antiviral therapeutics (S. Balasubramanian et al., Nat. Rev. Drug. Discov., 2011, 10, 261-275; M. Metifiot et al., Nucleic Acids Res., 2014, 42, 12352-12366). The core G4 unit is the G-tetrad, a cyclic planar arrangement of four guanines linked through Hoogsteen-type hydrogen bonds. Stacking of two or more G-tetrads generates a G-quadruplex structure, which is further stabilized by cations hosted in the central cavity of the G4. G4s exhibit a marked structural polymorphism, which depends on the number of strands involved in the structure and their relative orientation, on the types of linking loops, on the syn/anti conformation of the guanine residues and on the nature of the associated metal cations (G.W. Collie et al., Chem. Soc. Rev., 2011, 40, 5867-5892).

Recently, the presence of G4 structures has been unambiguously demonstrated in the genetic material of human cells, especially at telomeric level (G. Biffi et al., Nat. Chem., 2013, 5, 182-186). Telomeres are the ends of eukaryotic chromosomes, which protect chromosomal termini from unwanted degradation and guarantee proper replication. The 3' single-stranded overhang of the telomeric DNA, consisting of tandem repeats of G-rich sequences, can fold into peculiar G4 structures. In normal cells telomeres gradually shorten as a consequence of the end-replication problem - DNA polymerase is not able to replicate the chromosome up to its termination - and, on reaching critically short lengths, cells enter in replicative senescence, and ultimately in apoptosis. Conversely, in about 80-85% of cancer cells the enzyme telomerase, which provides the main mechanism of telomere lengthening, counterbalances the progressive shortening of the telomere, making them immortal (J. Bidzinska et al., Molecules, 2013, 18, 12368-12395). In this frame, telomeric G4s emerged as an attractive target in anticancer strategy since they are not recognized by telomerase, thereby inhibiting telomere elongation. Several experiments have fully demonstrated that known telomeric G4-ligands, as BRACO-19, RHPS4 and telomestatin, exert anticancer activity in vivo targeting telomeric DNA, thus validating their role as potential candidate drugs in anticancer strategies (E. Salvati et al., J. Clin. Invest., 2007, 117, 3236-3247). However, none of the above compounds, or other G4-ligands which proved to be very efficient in vitro anticancer agents, has advanced to clinical evaluation because of unfavourable pharmacokinetics and severe toxicities (Y.-X. Xiong et al., Eur. J. Med. Chem., 2015, 97, 538-551). Moreover, taking into account that the existing therapies are always associated with heavy side effects, due to the intrinsically poor selectivity of known anticancer/antiviral drugs, such as for example cisplatin and related analogues, the discovery of an efficient targeted therapy, causing limited-to-null toxicity, is urgently required (N.J. Wheate et al., Dalton Trans., 2010, 39, 8113-8127; D. Musumeci et al., Dalton Trans., 2016, 45, 8587-8600). Thus, considerable efforts are currently devoted to the design and identification of molecules able to selectively target peculiar DNA structures, discriminating duplex DNA. To be effective, this huge impulse to synthesize new potential ligands has to be coupled with fast, reliable High Throughput Screening (HTS) methods.

Affinity chromatography is a powerful methodology for probing small molecule-biomolecule interactions; it consists of simple and efficient assays by immobilizing the molecule or, more frequently, the biomolecule on a solid support, thus allowing screenings of large libraries in a short time. This technique is largely applied to select bioactive compounds from mixtures as, for example, is the case of aptamers obtained through SELEX methodology (C. Platella et al., Biochim. Biophys. Acta-Gen. Subj., 2017, 1861, 1429-1447).

In the search of HTS methods enabling a rapid and efficient identification of candidate anticancer drugs, recently a highly reproducible affinity chromatography-based method for the identification of putative G4-ligands has been described (D. Musumeci et al., Anal. Chem., 2014, 86, 4126-4130).

The Oligo Affinity Support (OAS), a commercially available polystyrene/polyethyleneglycol copolymer, functionalized with a DMT-protected adenosine by the adenine exocyclic amino group (Glen Research Corporation, Sterling, VA, USA), has been selected to realize the binding assay. The OAS resin was hence used for the assembly of the 26-mer ^{5'}(TTAGGG)₄TT^{3'} sequence (tel26), a truncation of human telomeric DNA, able to fold, under proper conditions, into a unimolecular G4. Due to its peculiar functionalization, the final aq. ammonia treatment fully deprotected the oligomer without detaching it from the support (http://www.glenresearch.com/Technical/TB_OAS.pdf).

The above method was optimized on known G4 and non-G4 ligands. Cationic porphyrin 5,10,15,20-tetrakis(1-methyl-4-pyridinio)porphyrin (TMPyP4), thiazole orange (TO), two acridine derivatives, i.e. 3,11-difluoro-6,8,13-trimethyl-8H-quino[4,3,2-kl]acridiniummethosulfate (RHPS4) and acridine-9-carboxylic acid (9-Acr-COOH), and two pyrrole-containing polyamide analogues, i.e. netropsin and distamycin, were analyzed (S. Neidle, FEBS J., 2010, 277, 1118-1125; B. Pagano et al., J. Nucleic Acids, 2010, doi: 10.4061/2010/247137; B. Pagano et al., Biochimie, 2008, 90, 1224-1232; R. Ferreira et al., Molecules, 2012, 17, 7067-7082; D. Monchaud et al., Biochimie, 2008, 90, 1207-1223; M.-K. Cheng et al., J. Med. Chem., 2008, 51, 963-975; L.A. Marky et al., Proc. Natl. Acad. Sci. USA, 1987, 84, 4359-4363). After the optimization, the binding assay, named G4-OAS (G4 on Oligo Affinity Support), was applied to the screening of a focused library of 60 small organic molecules, selected as potential G4 groove binders through a ligand-based virtual screening (VS). G4 groove binders are generally expected to exhibit higher discrimination ability in the recognition of G4 vs. duplex DNA than end-stackers, thus in principle assuring higher selectivity and consequently lower toxicity in vivo. For each of the 60 VS-selected molecules, the absence of unspecific binding on the nude OAS support was verified first, and, subsequently, the ability to interact with the G4-functionalized support. For each ligand, a stock solution was prepared by dissolving a weighed amount of the solid compound in pure DMSO. A known volume was withdrawn from the stock solution, so to obtain a 60 µM ligand solution. For each tested ligand, all the binding assays were carried out in triplicate, always using a freshly prepared 60 µM ligand solution. The general procedure adopted for the binding assays was as follows: a weighed amount of the resin (5.0 mg) was left in contact with 300 µl of the 60 µM ligand solution in a polypropylene column (4 ml capacity, Alltech) equipped with a PTFE frit (10 µm), a stopcock and a cap. After 4 min swelling on a vibrating shaker, the resin was eluted with defined volumes of a washing solution (50 mM KCl/20% DMSO) and all the eluted fractions were separately analysed by UV measurements. The composition of the washing solution was optimized on the basis of: solubility of the tested ligands, minimization of unspecific interactions of the ligands with the polystyrenic resin, capacity of the tel26 sequence to form stable G4 structure under the used conditions. From UV measurements of the fractions eluted from the nude OAS, the amount of ligand unspecifically adsorbed on the resin was evaluated. In turn, UV analysis of the fractions recovered from tel26-OAS allowed estimating the amount of ligand bound to the resin carrying the G4-forming strand. In all cases, the amount of bound ligand was calculated by subtracting the ligand eluted upon washing, derived by direct UV measurements, from the ligand amount initially loaded on the resin. Moreover, as further control, the direct measurement of the bound ligand was obtained by treating the resin with a releasing solution (2.5 M CaCl₂/15% DMSO in water or pure DMSO), followed by UV analysis of the eluted fractions. The amounts of bound ligands thus measured were in good agreement with those previously determined as a difference with respect to the unbound amounts. The composition of the releasing solution was optimized on the basis of its ability to obtain a fast and quantitative recovery of the bound ligand, which, in the case of tel26-OAS, was realized by efficient unfolding of the G4. In order to allow the correct G4 refolding after the treatment, and thus reuse the same batch of tel26-OAS for subsequent binding assays, the resin was resuspended in the washing solution and then subjected to an annealing procedure, by taking the resin at 75 °C for 5 minutes, followed by slow cooling at room temperature. High temperatures, such as annealing procedures at 85-95 °C, were avoided to prevent the detachment of the oligonucleotide from the resin. In all cases, UV controls showed that only 0.5-1.0% nucleotide material was released upon these thermal treatments. The reversibility of the process of folding/unfolding of the G4 allowed effectively recycling the resin; thus, a large number of binding assays could be performed on the same batch of resin without losing in efficiency and reliability of the experiments. Typically, a single batch of resin of 5.0 milligrams was used for the screening of at least 10 ligands, with each ligand analysed in triplicate, so to reach a total number of 30 experiments, before being discarded. The above assay allowed the identification of 7 out of the 60 compounds, which were then studied in solution using UV, CD, NMR and fluorescence spectroscopy in their interaction with the G4-forming human telomeric DNA sequence. Solution studies provided further evidence that the selected species were able to interact with the model telomeric G4 with good affinity, preferentially recognizing the G4 over duplex DNA. Furthermore, biological assays on human tumour cells demonstrated that 3 out of the 7 tested compounds were able to effectively induce a DNA damage response at telomeres, even at 1 µM concentration (D. Musumeci et al., Biochim. Biophys. Acta-Gen. Subj., 2017, 1861, 2017, 1341-1352.).

Most known binders of secondary structure-forming oligonucleotides, such as duplex and G-quadruplex, share a common planar aromatic core, necessary to realize π-π stacking interactions with the nucleobases, resulting in stable target-ligand complexes (Murat et al., Chem. Soc. Rev., 2011, 40, 5293-5307; Y. Xu et al., Chem. Soc. Rev., 2011, 40, 2719-2740).

Two-step processes to prepare supports for affinity chromatography, in which the oligonucleotide is modified introducing a terminal reactive functional group and then condensed with a suitably activated support, are known in the art (E.J. Devor et al., Integrated DNA Technologies, 2005; C.J. Larson et al., Nucleic Acids Res., 1992, 20, 3525).

The above prior art presents several drawbacks. The G4-OAS assay suffers from a major limitation resulting from the chemical nature of the OAS support. Indeed, around one third of the 60 VS-selected molecules showed strong unspecific binding on the nude OAS resin, due to stacking interactions between the polystyrenic resin and the aromatic cores of the ligands and was therefore discarded, making impossible the acquisition of information on their binding to tel26-OAS.

The linker disclosed in E.M. Southern et al., Support-bound oligonucleotide, US5436327A, 1989, is not stable to the aqueous ammonium hydroxide treatment commonly used, after the oligonucleotide elongation, for its deprotection also at the level of the nucleobases, required to be in a fully deprotected form in solid phase hybridization assays. Indeed, only 19% and 28% of a short oligonucleotide remained attached to controlled pore glass (CPG) and ballotini glass beads, corresponding to a final functionalization of 3.6 and 0.012 µmol/g, respectively, after standard ammonia treatment for 15 hours at 55 °C, necessary to achieve complete oligonucleotide deprotection.

Aiming at synthesizing G4-selective ligands with enhanced activity/toxicity ratio, a convenient synthetic strategy could be to start from known lead compounds featured by a suitable aromatic core and then functionalize them with different decorations. In this way, the stabilizing stacking interactions with the target can be preserved, and an improved selectivity achieved, by exploiting additional, specific electrostatic interactions and/or hydrogen bonds realized by the decorations with the target grooves. Unfortunately, many known binders with planar aromatic cores typically show strong unspecific binding with polystyrene-based resins, such as the ones used in the G4-OAS assay.

### Closest prior art

It is known in the art a solid support of controlled pore glass with a linker coupling a spacer to the first nucleoside via a succinate linkage used for the synthesis of oligonucleotides (S. Beaucage et al., , Tetrahedron, 1992, 48, 2223-2311).

It is also known a linker for the synthesis of oligonucleotides on glass supports, useful in solid phase hybridization assays, comprising a hexaethylene glycol spacer, bound to the glass via a glycidoxypropylsilane, terminating in a primary hydroxyl group that served as starting point for the oligonucleotide synthesis (U. Maskos et al., Nucleic Acids Res., 1992, 20, 1679-1684).

US patent N. US2006/149046 discloses a process for the synthesis of oligonucleotides which involves a solid support with a linker connecting the solid phase of long chain alkylamine (LCAA)/controlled pore glass (CPG) to the first nucleoside wherein the linker is a succinyl moiety.

### Technical problem

In view of the prior art, there is a strong felt need to provide supports, different from the commercially available OAS, being chemically inert and properly functionalized to elongate a secondary structure-forming oligonucleotide for affinity chromatography-based binding assays.

The inventors of the present invention developed a strategy to synthesize a universal glass support in which the first nucleoside is attached through the nucleobase via a stable hexaethylene glycol spacer, intended for the on-line solid phase synthesis of oligonucleotides and subsequent affinity chromatography assays based on oligonucleotides, overcoming the drawbacks connected with the use of polystyrenic resins.

The distinguishing feature is the linker made of hexaethylene glycol as a spacer and one nucleoside wherein the hexaethylene glycol is bound to the nucleoside through the nucleobase via a C-N or C-0 bond.

The technical effect of the above distinguishing feature is that in the linker the C-N and C-O bonds are resistant to the deprotecting steps, involving a treatment with an ammonium hydroxide solution, of the oligonucleotide, which is therefore left attached to the solid support.

The support could be useful for the High Throughput Screening (HTS) of putative ligands selectively recognizing secondary structure-forming oligonucleotides.

Since the direct synthesis of oligonucleotides on the solid support used for the affinity chromatography-based binding assays offers clear advantages, reducing time and costs of the whole process, the inventors of the present invention purposively selected a proper solid support and a suitable linker to obtain a functionalized support allowing the correct elongation of the oligonucleotide and, after protecting groups removal, the successive affinity chromatography-based binding assays.

Aiming at speeding the search for effective drugs in targeted therapies, the choice of the solid support and linker on which you can elongate the oligonucleotide is particularly relevant to obtain efficient oligonucleotide-functionalized matrices for affinity chromatography assays which allow expanding the chemical space of potential ligands as well as discarding false positives.

### Object of the invention

With reference to the attached claims and the following detailed description, the above technical problem is solved by providing a process for the preparation of a functionalized support made of a solid support being controlled pore glass (CPG) and a linker made of a spacer and one nucleoside wherein the spacer is hexaethylene glycol comprising the following steps:
a) Mixing controlled pore glass (LCAA-CPG) with 4,4'-dimethoxytrityl-hexaethylene glycol-COOH (DMT-HEG-COOH) in the presence of an activator of the carboxylic functional group to obtain a CPG solid support functionalized with hexaethylene glycol as the spacer bound to the support through an aliphatic amide function and protected at the terminal hydroxyl moiety with a 4,4'-dimethoxytrityl (DMT) protecting group;
b) Removal of the DMT protecting group from the product of step a) by acidic treatment;
c) Incorporation of a sugar-protected nucleoside in the product of step b) through a Mitsunobu reaction using triphenylphosphine and an azodicarboxylate as activators to obtain a CPG solid support functionalized with a nucleoside, wherein the nucleoside is attached to the CPG solid support through the nucleobase via a C-N or C-O bond so that, after the acidic treatment allowing the removal of the 4,4'-dimethoxytrityl (DMT) protecting group at 5' end of the sugar-protected nucleoside, the 5'-position is available for oligonucleotide elongation.

A further object is also the functionalized support made of a solid support being controlled pore glass (CPG) and a linker made of a spacer and one nucleoside wherein the spacer is hexaethylene glycol which is prepared by the above process.

It is also an object of the present invention a method for the synthesis of support-bound fully deprotected oligonucleotides.

It is also an object of the present invention the use of the functionalized support as a binding support for affinity chromatography.

It is also an object of the present invention the use of the functionalized support for the synthesis of support-bound fully deprotected oligonucleotides.

### Brief Description of the Drawings

Figure 1 shows the circular dichroism spectra of SEQ.ID. NO.:1 and bimolecular Dickerson dodecamer.
Figure 2 shows the HPLC profile of the eluate relative to ammonium hydroxide/methylamine (AMA) deprotection of the hairpin duplex-bound support 2.

### Detailed Description of the Invention

### Definitions

Within the meaning of the present invention functionalized support means the stationary phase carrying specific chemical groups, made of a chemically inert solid support and a linker made of a spacer connecting reactive chemical groups with the solid support.

Within the meaning of the present invention controlled pore glass (CPG) means a non-crystalline amorphous solid (glass) including nanometric or micrometric pores produced from a borosilicate base. Preferred CPG is Long Chain Alkylamine-CPG (LCAA-CPG).

Within the meaning of the present invention the spacer is hexaethylene glycol bound to the solid support by an aliphatic amide linkage realized with the terminal amine of LCAA-CPG through the methods of peptide coupling (S.B.H. Kent, Ann. Rev. Biochem., 1984, 57, 957-989; E. Atherton and R.C. Sheppard, Solid Phase Peptide Synthesis: a Practical Approach, 1989, IRL Press, Oxford, England; M. Bodanszky, Principles of Peptide Synthesis. Reactivity and Structure: Concepts in Organic Chemistry, 2012, Springer Science & Business Media, New York, NY, 9-52).

Within the meaning of the present invention reactive chemical groups are nucleosides which are attached to the spacer through their nucleobase so to have the 5' position available for oligonucleotide elongation.

Within the meaning of the present invention nucleoside consists of a nucleobase and a five-carbon sugar, which preferably does not contain a purine, such as adenine and guanine, therefore contains a pyrimidine, such as cytosine, thymine and uracil.

Within the meaning of the present invention activator of the carboxylic functional group means a nucleophile which rapidly reacts with the carboxylic group introducing onto the electrophilic carbon a better leaving group than OH⁻ (hydroxide ion) and thus facilitating the conversion of the carboxylic acid into its derivatives, such as esters and amides. All the compounds promoting peptide coupling are generally activators of the carboxylic function.

Within the meaning of the present invention compound promoting peptide coupling means a compound temporarily attaching a carboxylate group and forming a highly electrophilic intermediate, rendering the nucleophilic attack by the terminal amino group of LCAA-CPG support more efficient. Compounds promoting peptide coupling can be selected from the group consisting of N,N'-dicyclohexylcarbodiimide (DCC), N,N'-diisopropylcarbodiimide (DIC), N-cyclohexyl,N'-isopropylcarbodiimide (DCI), N-(3-dimethylaminopropyl)-N'-ethylcarbodiimide hydrochloride (EDC), alone or mixed with 1-hydroxybenzotriazole (HOBt) or 1-hydroxy-7-aza-1H-benzotriazole (HOAt).

Most preferred compound promoting peptide coupling is N,N'-dicyclohexylcarbodiimide (DCC) in the presence of the activator 1-hydroxybenzotriazole (HOBt).

Within the meaning of the present invention aliphatic amide function means a functional group including the NH-CO (amide) linkage in which the N atom is attached to an alkyl group (R-NH-CO-).

Within the meaning of the present invention terminal hydroxyl moiety means a group consisting of the OH (hydroxyl) function attached at the extremity of a given molecular structure.

Within the meaning of the present invention protecting group means a group which is introduced into a molecule by chemical modification of a functional group to obtain chemoselectivity in a subsequent chemical reaction; in oligonucleotide synthesis a suitable protecting group for the 5'-hydroxyl position can be selected from the group consisting of: 4-monomethoxytrityl (MMT); 4,4'-dimethoxytrityl (DMT).

The most preferred 5'-OH protecting group is 4,4'-dimethoxytrityl (DMT).

Within the meaning of the present invention acidic treatment means solution conditions wherein pH is that of a 3% trichloroacetic acid solution in dichloromethane or of a 80% acetic acid solution in water.

Within the meaning of the present invention sugar-protected nucleoside means a nucleoside in which the OH (hydroxyl) groups on the five-carbon sugar are blocked with protecting groups.

Most preferred sugar-protected nucleoside is 5'-O-DMT, 3'-O-acetyl-thymidine.

Within the meaning of the present invention Mitsunobu reaction means an organic reaction that converts an alcohol into a variety of functional groups, using as activators triphenylphosphine and an azodicarboxylate such as diethyl azodicarboxylate (DEAD) or diisopropylazodicarboxylate (DIAD) (T.Y. But et al., Chem. Asian J., 2007, 2, 1340-1355; E. Quezada et al., Helv. Chim. Acta, 2010, 93, 309-313).

Within the meaning of the present invention standard phosphoramidite activator or standard activator of the phosphoramidite monomer means a slightly acidic compound able to protonate the nitrogen atom of the phosphoramidite moiety in the nucleotide building blocks commonly used in solid phase oligonucleotide synthesis, allowing it to react with the terminal 5'-hydroxyl group of the extending oligonucleotide chain.

Object of the present invention is a process for the preparation of functionalized support made of a solid support being controlled pore glass (CPG) and a linker made of a spacer and one nucleoside wherein the spacer is hexaethylene glycol comprising the following steps:
a) Mixing controlled pore glass (LCAA-CPG) with 4,4'-dimethoxytrityl-hexaethylene glycol-COOH (DMT-HEG-COOH) in the presence of an activator of the carboxylic functional group to obtain a CPG solid support functionalized with hexaethylene glycol as the spacer bound to the support through an aliphatic amide function and protected at the terminal hydroxyl moiety with a 4,4'-dimethoxytrityl (DMT) protecting group;
b) Removal of the DMT protecting group from the product of step a) by acidic treatment;
c) Incorporation of a sugar-protected nucleoside in the product of step b) through a Mitsunobu reaction using triphenylphosphine and an azodicarboxylate as activators to obtain a CPG solid support functionalized with a nucleoside, wherein the nucleoside is attached to the CPG solid support through the nucleobase via a C-N or C-O bond so that, after the acidic treatment allowing the removal of the 4,4'-dimethoxytrityl (DMT) protecting group at 5' end of the sugar-protected nucleoside, the 5'-position is available for oligonucleotide elongation.

A further object is also the functionalized support made of a solid support being controlled pore glass (CPG) and a linker made of a spacer and one nucleoside wherein the spacer is hexaethylene glycol, which is prepared by a process comprising the following steps:
a) Mixing controlled pore glass (LCAA-CPG) with 4,4'-dimethoxytrityl-hexaethylene glycol-COOH (DMT-HEG-COOH) in the presence of an activator of the carboxylic functional group to obtain a CPG solid support functionalized with hexaethylene glycol as the spacer bound to the support through an aliphatic amide function and protected at the terminal hydroxyl moiety with a 4,4'-dimethoxytrityl (DMT) protecting group;
b) Removal of the DMT protecting group from the product of step a) by acidic treatment;
c) Incorporation of a sugar-protected nucleoside in the product of step b) through a Mitsunobu reaction using triphenylphosphine and an azodicarboxylate as activators to obtain a CPG solid support functionalized with a nucleoside, wherein the nucleoside is attached to the CPG solid support through the nucleobase via a C-N or C-O bond so that, after the acidic treatment allowing the removal of the 4,4'-dimethoxytrityl (DMT) protecting group at 5' end of the sugar-protected nucleoside, the 5'-position is available for oligonucleotide elongation.

Controlled pore glass is the support of choice because of its chemical inertness and stability, which render it more convenient than polystyrene resins for oligonucleotide synthesis and affinity chromatography.

The hexaethylene glycol spacer is a long and flexible spacer, with good solubility properties in both apolar and polar solvents, which guarantees minimization of possible steric effects with the solid matrix during the oligonucleotide synthesis and binding assays on the support as well, also ensuring the appropriate flexibility and distance from the support of the anchored oligonucleotide, which can thus adopt its preferred secondary structure as if in solution.

In order to avoid undesired, non-specific interactions in binding assays, purine nucleosides, such as adenosine and guanosine, were not selected as first monomers to be attached onto the support, since their nucleobases can generate additional stacking interactions with both the tested ligand and the oligonucleotide sequence, somehow affecting its native conformation. On the contrary, OAS resin, known in the art, presents a purine nucleoside as the first monomer.

Since the Controlled Pore Glass is functionalized with a hexaethylene glycol spacer, and the first monomer then incorporated by a Mitsunobu reaction is fully protected at the sugar moiety, the process allows attaching the nucleoside through the nucleobase, so to have the sugar 5'-position, after DMT removal, available for the successive oligonucleotide elongation.

Moreover, the conditions for the oligonucleotide-bound support deprotection are optimized so to achieve a suitable final functionalization, which is not too low, thus allowing the use of small amounts of support for the binding tests, nor too high, to avoid steric hindrance problems in the binding assays.

Preferably controlled pore glass (CPG) is Long Chain Alkylamine-CPG (LCAA-CPG).

Preferably the sugar-protected nucleoside is a pyrimidine nucleoside, more preferably the nucleoside is 5'-O-DMT-3'-O-acetyl-thymidine.

Preferably in step a) Controlled Pore Glass (CPG) and 4,4'-dimethoxytrityl-hexaethylene glycol-COOH (DMT-HEG-COOH) are mixed in 1:10 equivalent ratio.

Preferably in step b) acidic treatment is with 3% TCA in CH₂Cl₂.

Another object of the present invention is a method for the synthesis of fully deprotected oligonucleotides bound to a functionalized support made of a solid support being controlled pore glass (CPG) and a linker made of a spacer and one nucleoside wherein the spacer is hexaethylene glycol, comprising the steps of:
a) Mixing controlled pore glass (LCAA-CPG) with 4,4'-dimethoxytrityl-hexaethylene glycol-COOH (DMT-HEG-COOH) in the presence of an activator of the carboxylic functional group to obtain a CPG solid support functionalized with hexaethylene glycol as the spacer bound to the support through an aliphatic amide function and protected at the terminal hydroxyl moiety with a 4,4'-dimethoxytrityl (DMT) protecting group;
b) Removal of the DMT protecting group from the product of step a) by acidic treatment;
c) Incorporation of a sugar-protected nucleoside in the product of step b) through a Mitsunobu reaction using triphenylphosphine and an azodicarboxylate as activators to obtain a CPG solid support functionalized with a nucleoside, wherein the nucleoside is attached to the CPG solid support through the nucleobase via a C-N or C-O bond so that, after the acidic treatment allowing the removal of the 4,4'-dimethoxytrityl (DMT) protecting group at 5' end of the sugar-protected nucleoside, the 5'-position is available for oligonucleotide elongation;
d) Coupling of the 5'-position available for oligonucleotide elongation (5'-OH), as obtained in step c), with a nucleoside 3'-O-(2-cyanoethyl)-N,N-diisopropylphosphoramidite monomer in the presence of a standard phosphoramidite activator to obtain a phosphite triester linkage;
e) Capping of 5'-OH groups on the solid support which did not react in step d) by using a mixture of acetic anhydride and N-methylimidazole in pyridine;
f) Oxidation of the phosphite triester linkage as obtained in step d) to phosphate triester moiety by using a solution of I₂/H₂O/pyridine in THF;
g) Repetition of the steps from d) to f) for a number of times corresponding to the number of nucleotides contained in the oligonucleotide sequence to be obtained;
h) Deprotection, at the end of the oligonucleotide chain assembly obtained by step g), of the oligonucleotide bound to the functionalized support by treatment with an ammonium hydroxide solution to obtain the fully deprotected oligonucleotide.

The oligonucleotide elongation is based on phosphoramidite chemistry as disclosed in S.L. Beaucage et al., Tetrahedron, 1992, 48, 2223-2311.

Preferably in step d) the standard phosphoramidite activator of the nucleoside phosphoramidite monomers is selected from the group consisting of: 1H-tetrazole, 5-ethylthiotetrazole (ETT), 4,5-dicyanoimidazole (DCI).

Preferably in step h) the ammonium hydroxide solution is 30% ammonium hydroxide/40% methylamine 1:1, v/v (AMA).

Preferably in step h) the deprotection of the oligonucleotide bound to the functionalized support by treatment with ammonium hydroxide solution to obtain the fully deprotected oligonucleotide is carried out with 30% ammonium hydroxide/40% methylamine 1:1, v/v (AMA) at room temperature for 2 hours.

In the method of the present invention the linkage of the first nucleoside to the hexaethylene glycol spacer occurs through the nucleobase, by means of a chemically stable C-N or C-O bond, so that, once the oligonucleotide chain elongation is completed, the oligonucleotides can be fully deprotected without detaching them from the solid support.

This feature distinguishes the support-bound oligonucleotides, as obtained in the present invention, from the oligonucleotides linked to the solid support, as disclosed in the prior art: linked by standard succinyl linkers (S.L. Beaucage et al., Tetrahedron, 1992, 48, 2223-2311; K. Arar, Methods and compositions for the tandem synthesis of two or more oligonucleotides on the same solid support, US2006/0149046A1, 2006), or by glycol-phosphate moieties (U. Maskos et al., Nucleic Acids Res., 1992, 20, 1679-1684). The oligonucleotides linked to the solid support, of the prior art as above disclosed, undergo hydrolysis in aqueous basic solution thus completely releasing in solution the deprotected oligonucleotides at the end of the synthesis.

On the contrary, in the method of the present invention the linker is stable in all reaction conditions and upon thermal treatments up to 75 °C, and is stable in the conditions of the deprotection steps of the oligonucleotide, guaranteeing a proper functionalization for the successive affinity chromatography-based binding assays.

The functionalized support can be used as a binding support for affinity chromatography and for affinity chromatography-based assays.

The functionalized support can be used as a binding support for affinity chromatography-based screenings of libraries of putative ligands of secondary structure-forming oligonucleotides.

It is also an object of the present invention the use of the functionalized support for the synthesis of support-bound fully deprotected oligonucleotides.

On the functionalized support of the present invention a large variety of biologically relevant DNA and/or RNA sequences, human or viral, can be elongated, using standard phosphoramidite chemistry by automated DNA/RNA synthesizer.

On the functionalized support of the present invention PNA or DNA/PNA mixed sequences can be elongated, introducing an additional linker providing a terminal reactive amino (NH₂) group such as 6-(4-monomethoxytritylamino)hexyl-(2-cyanoethyl)-(N,N-diisopropyl)-phosphoramidite and then using standard Fmoc/Bhoc chemistry for the PNA assembly on an automated PNA synthesizer.

On the functionalized support of the present invention oligonucleotide sequences as controls, e.g. hairpin-duplex or duplex structures, can be elongated for the evaluation of the recognition specificity of the tested ligands in the affinity chromatography-based binding assays.

In a preferred embodiment of the present invention the functionalized support of the present invention is prepared by mixing standard Long Chain Alkylamine (LCAA-CPG) with DMT-HEG-COOH in 1:10 equivalent ratio in DMF, as described below in detail. In this way, the LCAA linker is further elongated by addition of a hexaethylene glycol spacer. Indeed, the insertion of this long and flexible spacer, with good solubility properties in both apolar and polar solvents, guarantees minimization of possible steric effects with the solid matrix during the oligonucleotide synthesis and binding assays on the support as well. This spacer also ensures the appropriate flexibility and distance from the support of the anchored oligonucleotide, which can thus adopt its preferred secondary structure as if in solution. The obtained functionalized support, after standard DMT removal by acidic treatment (3% TCA in CH₂Cl₂) is reacted with 5'-O-DMT, 3'-O-acetyl-thymidine; the nucleoside is attached through the nucleobase, so to have, after standard 5'-DMT removal, the sugar 5'-position available for the successive oligonucleotide elongation.

### Examples

Synthesis of DMT-HEG-COOH: 450 mg (0.77 mmol) of DMT-HEG-OH (commercially available from Berry & Associates) were reacted with a mixture of 19 mg (0.12 mmol) of 2,2,6,6-tetramethyl-1-piperidinyloxy (TEMPO), 20 mg (0.17 mmol) of KBr, 46 mg (0.17 mmol) of tetrabutylammonium chloride, 1 ml of NaHCO₃ (saturated solution), 2.5 ml of NaClO and 1 ml of NaCl (satd. solution) at r.t. for 4 h. The resulting product (DMT-HEG-COOH) was purified by flash chromatography on a silica gel column eluted with increasing amounts of CH₃OH in CH₂Cl₂, from 5% to 10%, containing 1% of triethylamine (yield = 62%). The identity of the purified product was confirmed by ¹H NMR (C₆D₆, 400 MHz) : δ 8.11 (br, 1H, COOH), 7.43-6.48 (complex signals, 13H, DMT aromatic protons), 3.88 (s, 2H, CH₂CO), 3.34-3.06 (complex signals, 20H, 5x OCH₂CH₂), 3.21 (s, 6H, 2x OCH₃).

Functionalization of standard CPG with the hexaethylene glycol spacer DMT-HEG-COOH: 155 mg of LCAA-CPG 1000 Å (0.069 meq/g, 10.7 µmol; Link Technologies, Bellshill, UK) were reacted with a mixture of 64 mg (107 µmol) of DMT-HEG-COOH, 22 mg (107 µmol) of N,N'-dicyclohexylcarbodiimide (DCC) and 14.5 mg (107 µmol) of 1-hydroxybenzotriazole (HOBt) in 500 µL of anhydrous DMF at r.t. overnight. After exhaustive washings with DMF, CH₃OH, AcOEt, n-hexane and CH₂Cl₂, the support was taken to dryness under reduced pressure. DMT tests performed on dried and weighed samples of the resulting support provided the incorporation yield of DMT-HEG-COOH onto the CPG resin, which resulted to be 74% (0.051 meq/g) . A capping reaction with acetic anhydride in pyridine (1:1, v/v) was then performed at r.t. for 4 h to block the unreacted amino groups. After exhaustive washings with CH₂Cl₂, CH₃OH, and again CH₂Cl₂, the DMT test, performed on dried and weighed samples, gave a functionalization of 0.051 meq/g, in agreement with the previous value. Finally, the DMT protecting group was removed by treatment with 3% TCA in CH₂Cl₂ thus obtaining support 1, as reported in the following reaction scheme.

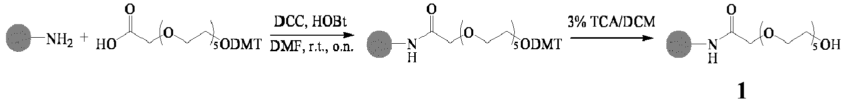

Synthesis of 5'-0-DMT, 3'-O-acetyl-thymidine: 94 mg of 5'-O-DMT-thymidine (Sigma-Aldrich) were reacted with acetic anhydride in pyridine (1:1, v/v) at r.t. for 2 h. The resulting product (5'-O-DMT, 3'-O-acetyl-thymidine) was taken to dryness under reduced pressure, dissolved in CH₂Cl₂ and purified by water/CH₂Cl₂ extraction. The identity of the purified product was confirmed by ¹H NMR (C₆D₆, 400 MHz) : δ 8.81 (br, 1H, NH T residue), 7.62-6.82 (complex signals, 13H, DMT aromatic protons), 7.51 (s, 1H, H-6 T residue), 6.64 (t, 1H, H-1' T residue), 5.42 (s, 1H, H-4' T residue), 4.01 (s, 1H, H-3' T residue), 3.54 (s, 2H, H-5' T residue), 3.36 (s, 6H, 2x OCH₃), 2.18 (d, 2H, H-2' T residue), 1.59 (s, 6H, CH₃ T residue and CH₃CO).

Functionalization of support 1 with 5'-O-DMT, 3'-O-acetyl-thymidine: 118 µL (0.75 mmol) of diethylazadicarboxylate (DEAD) were added to a mixture of 145 mg (0.051 meq/g, 7.4 µmol) of support 1, 87 mg (0.15 mmol) of 5'-0-DMT, 3'-O-acetyl-thymidine and 122 mg (0.47 mmol) of triphenylphosphine (PPh₃) in 1.5 ml of THF at r.t. overnight, according to the following reaction scheme. 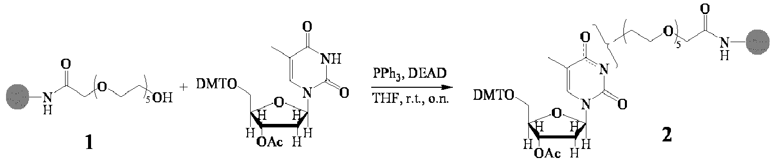 After exhaustive washings with CH₂Cl₂, CH₃OH, CH₃CN and again with CH₂Cl₂, the resulting support 2 was dried under reduced pressure.

Incorporation yield of 5'-O-DMT, 3'-O-acetyl-thymidine onto support 1 was 45% (0.023 meq/g), as determined by DMT tests performed on dried and weighed samples of support 2. Reiteration of this reaction did not allow increasing the support functionalization. A capping reaction with acetic anhydride in pyridine (1:1, v/v) was then performed at r.t. for 4 h to block unreacted hydroxyl groups on the support. After exhaustive washings with CH₂Cl₂, CH₃OH, and again CH₂Cl₂, the DMT test, performed on dried and weighed samples, gave a functionalization of 0.023 meq/g, in agreement with previous data.

Monitoring of possible unspecific binding on the nude CPG support: aiming at checking the absence of possible interferences of our modified CPG support in the successive binding assays, 4.35 mg of support 2 (100 nmol), synthesized as described above, were treated with 3% TCA in CH₂Cl₂ to remove the DMT protecting group. After exhaustive washings with CH₂Cl₂, CH₃OH, and again CH₂Cl₂, the support (hereafter named nude CPG) was taken to dryness and then left in contact with 300 µl of a 60 µM solution (18 nmol) of different ligands, having either good or no affinity for G4 (distamycin, netropsin, resveratrol, RHPS4, TO, TMPyP4, 9-Acr-COOH) in a polypropylene column equipped with a PTFE frit. Using the same protocol adopted for the G4-OAS assay, after 4 min incubation on a vibrating shaker, the surnatant was allowed flowing through the support and the nude CPG was washed with several volumes of the washing solution. The eluted fractions were collected and analysed by UV measurements.

Some minor modifications were introduced to improve the initially described G4-OAS assay. Indeed, the composition of the washing solution was further optimised: instead of 100 mM KCl/15% DMSO or 50 mM KCl/20% DMSO, 50 mM KCl/10% DMSO/10% CH₃CH₂OH was here used. In particular, a small percentage of ethanol was introduced in the washing solution so to fully avoid undesired absorption of the tested ligands on the assay equipment, i.e. the polypropylene column and the PTFE frit. However, the total percentage of organic solvent was unaltered (20%) to guarantee the solubility of the tested ligands without perturbing the correct folding of the support-bound oligonucleotide. Moreover, upon varying the overall composition of the washing solution, but keeping constant the total percentage of organic solvent in the 15-20% range, no change was observed, within the experimental error, in the amounts of released/bound ligand.

The results of the binding assays on the ligands tested on the nude CPG are shown in Table 1, wherein the recovered ligands were obtained using the washing solution: 50 mM KCl/10% DMSO/10% CH₃CH₂OH, the amounts of recovered ligands are expressed as percentage of the quantity initially loaded on the support, the errors associated with the reported percentages are within ± 2%.

**Table 1**

| **Ligand** | **Recovered ligand from nude CPG (%)** | **Recovered ligand from nude OAS (%)** |
|---|---|---|
| Distamycin | 96 | 93 |
| Netropsin | 95 | 100 |
| Resveratrol | 98 | 74 |
| RHPS4 | 93 | 93 |
| TO | 92 | 90 |
| TMPyP4 | 85 | 97 |
| 9-Acr-COOH | 100 | 95 |
| 5B | 97 | 9 |
| 10B | 100 | 79 |
| 7E | 96 | 92 |
| 7F | 100 | 92 |

These data were compared with those obtained on the nude OAS. Remarkably, the ligands were generally recovered in higher amounts from nude CPG, requiring smaller volumes of the washing solution for their quantitative recovery compared to OAS resin. Furthermore, also a set of compounds from the 60 VS-selected molecules previously investigated on G4-OAS was tested on the nude CPG support. Although a significant reduction of unspecific binding to the solid support was observed for all the tested ligands, the best case was compound 5B. Contrarily to what observed on nude OAS support, for which compound 5B had shown high affinity, in the experiments with nude CPG complete absence of binding was found. Overall, these results proved that the novel support has low-to-null unspecific interactions with the tested model ligands, in contrast to previously used OAS.

Assembly and deprotection of the hairpin duplex-forming oligonucleotide covalently attached to the CPG support: in the search for effective drugs in targeted therapies, a major challenge is the identification of molecules able to selectively target peculiar or even unusual DNA structures, discriminating duplex DNA. Our previous researches have emphasized the need to use a duplex system, in association with the single strand system already in use in all our tests, as negative control in the affinity chromatography-based binding assays. Thus we here synthesized a duplex-functionalized solid support, allowing assessing the ability of putative ligands to discriminate non-canonical DNA conformations vs. duplex structures. To this purpose, we chose, for the elongation on support 2, SEQ ID NO: 1, able to fold into a unimolecular duplex under proper conditions. It consists of two Dickerson dodecamer tracts - the best well-known model of B-DNA duplex (H.R. Drew et al., Proc. Natl. Acad. Sci. USA, 1981, 71, 2179-2183) - connected by a TTT loop. The length and base composition of the loop were selected on the basis of the following observations: loops longer than 3 residues can promote the formation of Z-DNA unimolecular duplex structures;
among the loops containing 1, 2 and 3 residues, the highest thermal stability is observed in the case of a 3 residues loop (L.E. Xodo et al., Nucleic Acids Res., 1991, 19, 1505-1511);
the nucleobases present in the loop should minimally affect the binding of the putative ligands to the duplex; thus, pyrimidine nucleosides are preferred since additional stacking interactions can be produced by purines;
the nucleobases not requiring protecting groups (as is the case of thymines) make easier and speed up the final deprotection step in solid phase oligonucleotide syntheses.

In order to verify if the TTT loop somehow perturbs the conformation adopted by the Dickerson dodecamer duplex, experiments were carried out on suitable model systems in solution. In detail, circular dichroism spectra, in the conditions used for the binding assay, i.e. 50 mM KC1/10% DMSO/10% CH₃CH₂OH, were registered and compared for 2 µM solutions of SEQ ID NO.: 1 CGCGAATTCGCGTTTCGCGAATTCGCG
and the bimolecular Dickerson dodecamer.

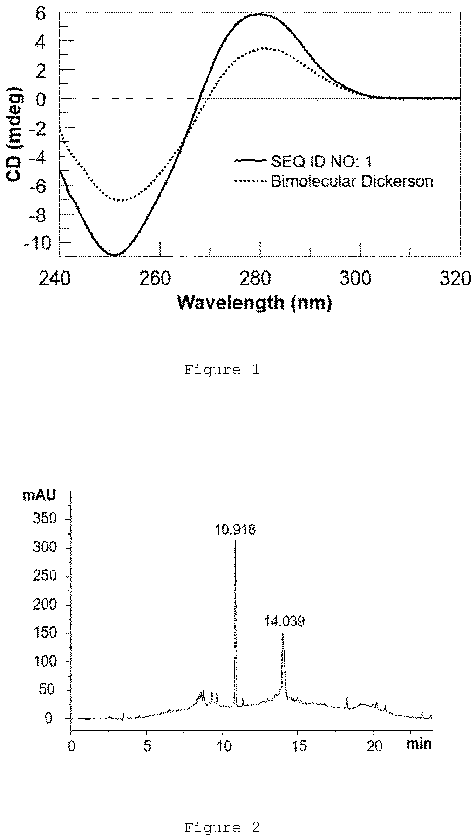

SEQ ID NO: 1 showed the typical CD features of a B-DNA conformation (Kypr et al., Nucleic Acids Res., 2009, 37, 1713-1725), with enhanced structuration compared to the bimolecular Dickerson dodecamer, as shown in figure 1.

CD-melting experiments were also performed, showing that the hairpin duplex (Tₘ = 73 °C) was more stable than the bimolecular duplex (Tₘ = 55 °C), with a ΔTₘ of 18 °C. These results validate SEQ ID NO: 1 as a good, stable B-DNA duplex model. It can be thus expected that this oligonucleotide, after annealing, folds into a stable hairpin-duplex system also on the support. This model system is much more convenient than previously synthesized OAS-linked single strand, then hybridized to its complementary strand so to obtain a bimolecular duplex on the same support. In fact, as previously demonstrated, such a duplex system is not stable in the conditions used for the successive binding assays, partially releasing the complementary strand in solution at each assay and thus providing only partially reliable results (D. Musumeci et al., Biochim. Biophys. Acta-Gen. Subj., 2017, 1861, 1341-1352).

Therefore, using standard phosphoramidite chemistry on an automated DNA/RNA synthesizer (1 µmol-scale, DMT-on mode, manual detachment), the oligonucleotide of sequence SEQ ID NO:1 was assembled on support 2. The coupling efficiency was monitored after each synthetic cycle by spectrophotometric measurements of the DMT cation, removed before each coupling step by standard acidic treatment with 3% TCA in CH₂Cl₂. Considering the number of couplings (27 bases) and the average yield per cycle of 99.2%, the overall yield was determined to be 80%, corresponding to a final functionalization of 0.018 meq/g. After 27 coupling cycles, aiming at completely deprotecting the oligonucleotide without detaching it from the solid support, two different deprotection protocols were tested. Two batches of oligonucleotide-bound support - still bearing the terminal 5' DMT group - were treated in parallel with 30% ammonium hydroxide (NH₄OH) at r.t. for 24 h, and with 30% ammonium hydroxide/40% methylamine 1:1, v/v (AMA) at r.t. for 2 h, respectively. The resulting supports were then washed several times with water until neutralization. To verify if the final basic treatment promoted partial detachment of the oligonucleotide from the solid supports, the eluates relative to NH₄OH and AMA deprotection were collected, taken to dryness, redissolved in H₂O and analysed by UV spectroscopy. In both cases, the presence of some nucleotide material was observed. This is probably due to silica instability under basic solutions; indeed, pure silica partially dissolves under these conditions (Y. Niibori et al., J. Nucl. Sci. Technol., 2000, 37, 349-357). The DMT test was then performed on dried and weighed samples of the two CPG batches, allowing the determination of the final functionalization for hairpin duplex-bound supports, which resulted to be 0.005 meq/g (30% oligonucleotide remained attached to the support) and 0.011 meq/g (60% oligonucleotide remained attached to the support) after the NH₄OH and AMA deprotection treatment, respectively. Taking for granted that the NH₄OH treatment, carried out under standard conditions, resulted in the full deprotection of the oligonucleotide bound to the support, with the aim of evaluating if also the AMA deprotection treatment went to completeness, we used an indirect test: we compared the experimental data for known duplex ligands obtained from our affinity chromatography-based binding assay on two oligonucleotide-bound supports having undergone the mentioned different deprotection methods. For all the tested ligands, the results were always in good agreement within the experimental error (see Table 3). Moreover, as a further control, the eluate relative to AMA deprotection was analysed by HPLC on a Phenomenex analytical C18 column (250 x 4 mm) using the gradient: 3-40% B over 15 min (A-0.1 M triethylammonium acetate, pH = 7.0; B-CH₃CN; flow rate: 1 ml/min). As shown in figure 2, upon AMA deprotection, only two major species were present in the eluate: one peak at 10.9 min retention time, i.e. the full-length oligonucleotide detached, which had accidentally lost its 5'-DMT protecting group; and one peak at 14.0 min, identified as the DMT-on, full-length oligonucleotide detached. Indeed, upon detritylation of the latter species, only one peak, with the same retention time of the first product, appeared in the HPLC chromatogram. Overall, these results - even if indirectly - proved that the 2 h AMA treatment guarantees complete deprotection for the oligonucleotide bound to support 2, in accordance with current protocols of oligonucleotide synthesis (http://www.glenresearch.com/Technical/Deprotection.pdf), moreover sensibly reducing the detachment of the synthesized oligonucleotide from the CPG support compared to the NH₄OH treatment.

Assembly and deprotection of the G4-forming oligonucleotide on the support: according to the previous example, oligonucleotide of sequence SEQ ID NO.: 2 TTAGGGTTAGGGTTAGGGTTAGGGTT, a truncation of human telomeric DNA, able to fold in proper conditions into a unimolecular G4, was assembled on support 2 using standard phosphoramidite chemistry (1 µmol-scale, DMT-on mode, manual detachment) on an automated DNA/RNA synthesizer. The coupling efficiency was monitored after each synthetic cycle by spectrophotometric measurements of the DMT cation, removed before each coupling step by standard treatment with 3% TCA in CH₂Cl₂. Considering the number of couplings (26 bases) and the average yield per cycle of 99.4%, the overall yield was determined to be 86%, corresponding to a final functionalization of 0.020 meq/g. After the 26 coupling cycles, aiming at fully deprotecting the oligonucleotide, the solid support was treated with 30% ammonium hydroxide/40% methylamine 1:1, v/v (AMA) at r.t. for 2 h. The resulting support was then washed with water until neutralization. DMT tests, performed on dried and weighed samples of the resin, allowed determining the final functionalization for the support-bound G-quadruplex-forming oligonucleotide, which was 0.012 meq/g after the AMA deprotection treatment.

Use of support-bound G4- and hairpin duplex for affinity chromatography-based binding assays: using the same protocol adopted for the G4-OAS assay, as previously described, a set of known G4 and non-G4-binding ligands (distamycin, netropsin, resveratrol, RHPS4, TO, TMPyP4, 9-Acr-COOH) were tested on the novel support 2 and the results compared with those obtained on the OAS support. 8.3 mg and 9.1 mg of, respectively, dried G4- and hairpin duplex-functionalized support obtained from 2 (G4-2 and hairpin duplex-2, 100 nmol of oligonucleotide each) were in parallel left in contact, in two different polypropylene columns, with 300 µl of the washing solution and subjected to an annealing procedure (75 °C for 5 min, followed by slow cooling). After 16 h at 4 °C, the supernatants on the resin were collected along with several washings and checked by UV analysis. The quantification of the detached oligonucleotide amounts was obtained by measuring at 85 °C the absorbance at λ=260 nm, using the molar extinction coefficients calculated for the unstacked oligonucleotide (M.J. Cavalluzzi et al., Nucleic Acids Res., 2004, 32, e13). From these analyses, we estimated that up to a maximum of 0.1-0.5% of nucleotide material was detached from the support after each annealing procedure. These results show that a CPG support is even more advantageous than polystyrene-based resins. Indeed, less oligonucleotide is detached upon thermal treatments, more binding assays can be performed on the same batch of support without losing in efficiency and reliability of the experiments. In this case, it could be possible to perform three times the number of binding assays than on the G4-OAS resin, for which the detachment of 0.5-1.0% of nucleotide material was previously observed after each annealing.

G4-2 and hairpin duplex-2 were then treated, analogously to the control nude CPG, with the ligand dissolved at 60 µM concentration (300 µl, 18 nmol) in the washing solution. After 4 min incubation, the supports were treated with the washing solution several times and the eluted fractions were collected and analysed by UV measurements. To allow the complete release in solution of the oligonucleotide-bound ligands, the supports were then eluted with the releasing solution. The amounts of G4-bound ligand measured on the G4-2 support were in general agreement with those determined for the G4-OAS support, as shown in Table 2. Moreover, the percentage of G4-bound ligand for OAS support was in some cases overestimated, e.g. for resveratrol, accounting for the unspecific interactions of the tested ligands with the nude OAS resin. The experimental data acquired on G4-2 support are much cleaner and more reliable than those obtained on G4-OAS. Remarkably, the ligands tested on the G4-2 and hairpin duplex-2 supports were recovered in higher amounts and required smaller volumes of releasing solution than G4-OAS. These results allowed recycling the same batch of resin without losing in efficiency and reliability for a larger number of binding tests than in the case of G4-OAS. Interestingly, these results showed that the novel synthesized support has less unspecific interactions with the tested model ligands than previously used polystyrenic G4-OAS.

Finally, the retention order observed for both G4-2 and hairpin duplex-2 supports, as shown in the following Tables 2 and 3, reflects the affinity trend of the tested ligands for the human telomeric G4 and the Dickerson duplex, confirming the general reliability of the support 2-based method.

**Table 2**

| | **Support 2** | | **OAS** | |
|---|---|---|---|---|
| **Ligand** | **G4-bound ligand (%)** | **Recovered ligand from G4-2 (%)** | **G4-bound ligand (%)** | **Recovered ligand from G4-OAS (%)** |
| Distamycin | 22 | 96 | 28 | 89 |
| Netropsin | 86 | 67 | 93 | 91 |
| Resveratrol | 4 | 96 | 22 | 96 |
| RHPS4 | 99 | 100 | 96 | 93 |
| TO | 97 | 92 | 98 | 88 |
| TMPyP4 | 100 | 98 | 99 | 90 |
| 9-Acr-COOH | 0 | 100 | 1 | 100 |

wherein G4-bound ligand (%) is calculated as a difference from the unbound ligand, recovered with the washing solution 50 mM KC1/10% DMSO/10% CH₃CH₂OH; recovered ligand from G4-2 (%) and recovered ligand from G4-OAS (%) are obtained using as releasing solution either 2.5 M CaCl₂/15% DMSO or 100% DMSO; the amounts of bound/recovered ligands are expressed as percentage of the quantity initially loaded on the support; the errors associated to the percentages are within ± 2%.

**Table 3**

| | **Support 2** | |
|---|---|---|
| **Ligand** | **Hairpin duplex-bound ligand (%)** | **Recovered ligand from Hairpin duplex-2 (%)** |
| Distamycin | 36 | 93 |
| Netropsin | 52 | 98 |
| Resveratrol | 1 | 99 |
| RHPS4 | 48 | 100 |
| TO | 51 | 96 |
| TMPyP4 | 50 | 99 |
| 9-Acr-COOH | 0 | 100 |

wherein hairpin duplex-bound ligand (%) was calculated as a difference from the unbound ligand recovered with the washing solution 50 mM KCl/10% DMSO/10% CH₃CH₂OH; recovered ligand from hairpin duplex-2 (%) was obtained using as releasing solution either 2.5 M CaCl₂/15% DMSO or 100% DMSO; the amounts of bound/recovered ligands are expressed as percentage of the quantity initially loaded on the support; the errors associated to the reported percentages are within ± 2%.

## Claims

1. Process for the preparation of a functionalized support made of a solid support being Controlled Pore Glass (CPG) and a linker made of a spacer and one nucleoside wherein the spacer is hexaethylene glycol comprising the following steps:
a) Mixing controlled pore glass (CPG) with 4,4'-dimethoxytrityl-hexaethylene glycol-COOH (DMT-HEG-COOH) in the presence of an activator of the carboxylic functional group to obtain a CPG solid support functionalized with hexaethylene glycol as the spacer bound to the support through an aliphatic amide function and protected at the terminal hydroxyl moiety with a 4,4'-dimethoxytrityl (DMT) protecting group;
b) Removal of the DMT protecting group from the product of step a) by acidic treatment;
c) Incorporation of a sugar-protected nucleoside in the product of step b) through a Mitsunobu reaction using triphenylphosphine and an azodicarboxylate as activators to obtain a CPG solid support functionalized with a nucleoside, wherein the nucleoside is attached to the CPG solid support through the nucleobase via a C-N or C-O bond so that, after the acidic treatment allowing the removal of the 4,4'-dimethoxytrityl (DMT) protecting group at 5'-end of the sugar-protected nucleoside, the 5'-position is available for oligonucleotide elongation.

2. Process according to claim 1 wherein Controlled Pore Glass (CPG) is Long Chain Alkylamine-CPG (LCAA-CPG).

3. Process according to claim 1 wherein the nucleoside is a pyrimidine nucleoside.

4. Process according to claim 3 wherein the nucleoside is 5'-O-DMT-3'-O-acetyl-thymidine.

5. Functionalized support made of a solid support being Controlled Pore Glass (CPG) and a linker made of a spacer and one nucleoside wherein the spacer is hexaethylene glycol, which is prepared by a process comprising the following steps:
a) Mixing controlled pore glass (LCAA-CPG) with 4,4'-dimethoxytrityl-hexaethylene glycol-COOH (DMT-HEG-COOH) in the presence of an activator of the carboxylic functional group to obtain a CPG solid support functionalized with hexaethylene glycol as the spacer bound to the support through an aliphatic amide function and protected at the terminal hydroxyl moiety with a 4,4'-dimethoxytrityl (DMT) protecting group;
b) Removal of the DMT protecting group from the product of step a) by acidic treatment;
c) Incorporation of a sugar-protected nucleoside in the product of step b) through a Mitsunobu reaction using triphenylphosphine and an azodicarboxylate as activators to obtain a CPG solid support functionalized with a nucleoside, wherein the nucleoside is attached to the CPG solid support through the nucleobase via a C-N or C-O bond so that, after the acidic treatment allowing the removal of the 4,4'-dimethoxytrityl (DMT) protecting group at 5'- end of the sugar-protected nucleoside, the 5'-position is available for oligonucleotide elongation.

6. Method for the synthesis of fully deprotected oligonucleotides bound to a functionalized support made of a solid support being controlled pore glass (CPG) and a linker made of a spacer and one nucleoside wherein the spacer is hexaethylene glycol, comprising the steps of:
a) Mixing controlled pore glass (LCAA-CPG) with 4,4'-dimethoxytrityl-hexaethylene glycol-COOH (DMT-HEG-COOH) in the presence of an activator of the carboxylic functional group to obtain a CPG solid support functionalized with hexaethylene glycol as the spacer bound to the support through an aliphatic amide function and protected at the terminal hydroxyl moiety with a 4,4'-dimethoxytrityl (DMT) protecting group;
b) Removal of the DMT protecting group from the product of step a) by acidic treatment;
c) Incorporation of a sugar-protected nucleoside in the product of step b) through a Mitsunobu reaction using triphenylphosphine and an azodicarboxylate as activators to obtain a CPG solid support functionalized with a nucleoside, wherein the nucleoside is attached to the CPG solid support through the nucleobase via a C-N or C-O bond so that, after the acidic treatment allowing the removal of the 4,4'-dimethoxytrityl (DMT) protecting group at 5' end of the sugar-protected nucleoside, the 5'-position is available for oligonucleotide elongation;
d) Coupling of the 5'-position available for oligonucleotide elongation (5'-OH), as obtained in step c) with a nucleoside 3'-O-(2-cyanoethyl)-N,N-diisopropyl phosphoramidite monomer in the presence of a standard phosphoramidite activator to obtain a phosphite triester linkage;
e) Capping of 5'-OH groups on the solid support which did not react in step d) by using a mixture of acetic anhydride and N-methylimidazole in pyridine;
f) Oxidation of the phosphite triester linkage as obtained in step d) to phosphate triester moiety by using a solution of I₂/H₂O/pyridine in THF;
g) Repetition of the steps from d) to f) for a number of times corresponding to the number of nucleotides contained in the target oligonucleotide sequence;
h) Deprotection, at the end of the oligonucleotide chain assembly obtained by step g), of the oligonucleotide bound to the functionalized support by treatment with an ammonium hydroxide solution, preferably 30% ammonium hydroxide/40% methylamine 1:1, v/v (AMA), to obtain the fully deprotected oligonucleotide.

7. Use of the functionalized resin of claim 5 as a binding support for affinity chromatography.

8. Use of the functionalized resin of claim 5 as support for the synthesis of support-bound fully deprotected oligonucleotides.
